# FASCICULE DE BREVET EUROPEEN

(11) **EP 2 644 564 B1**
(45) Date de publication et mention de la délivrance du brevet: **15.04.2015**
(21) Numéro de dépôt: 13305217.5
(22) Date de dépôt: 26.02.2013
(51) Int. Cl.: C01B 3/32, C01B 3/38

(54) **Réacteur de désulfuration temporaire permettant le prétraitement d'une charge hydrocarbonée avant vaporéformage en vue d'une production d'hydrogène**
Reaktor zur vorübergehenden Entschwefelung, der die Vorbehandlung eines Kohlenwasserstoffeinsatzmaterials vor der Dampfreformierung zur Wasserstofferzeugung ermöglicht
Temporary desulphurization reactor for pre-treatment of a hydrocarbon feedstock prior to steam reforming in order to produce hydrogen

(30) Priorité: 27.03.2012 FR 1200911
(43) Date de publication de la demande: 02.10.2013
(73) Titulaire: IFP Energies nouvelles, 92852 Rueil-Malmaison Cedex (FR)
(72) Inventeur: Giroudiere, Fabrice, 69530 ORLIENAS (FR); Thomas, Michel, 69003 LYON (FR); Nebois, Cedric, 69200 VENISSIEUX (FR)

(56) Documents cités:
- EP-A1- 1 236 495
- EP-A1- 1 780 256
- WO-A1-2011/077752
- WO-A1-2012/169199
- JP-A- 1 275 697
- JP-A- 2006 008 459
- JP-A- 2006 111 766
- JP-A- 2009 079 183
- JP-A- 2009 249 203
- US-A1- 2005 022 449

## Description

### DOMAINE DE L'INVENTION

La présente invention décrit un réacteur qu'on appellera par la suite réacteur de préparation dans le contexte d'un procédé de production d'hydrogène par vaporeformage de charges hydrocarbonées, comme par exemple du gazole, ou bien de l'éthanol dénaturé.

Les hydrocarbures (de type essence ou gazole par exemple) ou alcools (bio-éthanol par exemple) utilisés pour alimenter les unités de production d'hydrogène par vaporeformage (vaporeformage autotherme ATR suivi d'un déplacement à la vapeur WGS par exemple) contiennent généralement quelques ppm de soufre (typiquement 10 ppm) qu'il peut être économiquement viable de retirer par des procédés connus d'adsorption sur solide de type oxydes métalliques, éventuellement à l'état partiellement réduit.

Ce peut-être par exemple des solides à base de nickel passivé sur alumine ou silice-alumine (par exemple le solide adsorbant AxTrap-405 d'Axens).

Les unités de production d'hydrogène, notamment lorsqu'elles sont de petite taille (typiquement 100 Nm³/h d'H₂ produit) peuvent être situées en dehors de tout complexe pétrochimique, par exemple dans une station service ou un bâtiment isolé, notamment lorsque l'hydrogène est utilisé pour alimenter des piles à combustibles ou pour des applications telles que l'industrie du verre ou l'industrie électronique. Ce peut également être des unités destinées à être embarquées (camions, bateaux, etc.).

Le procédé de production d'hydrogène par vaporeformage dans lequel s'inscrit la présente invention fait appel en particulier à un éthanol dit "dénaturé" qui comprend un certain pourcentage de soufre, car cette dénaturation est généralement obtenue par adjonction d'une faible quantité d'essence porteuse de soufre. Or ce soufre, même à des concentrations très faibles de l'ordre de la dizaine de ppm, constitue un poison pour le catalyseur mis en oeuvre dans le réacteur de vaporeformage. Il peut néanmoins être facilement étendu à d'autres types de charges comme des essences ou des gazoles qui contiennent, après hydrotraitement catalytique poussé, encore quelques traces de composé soufrés, essentiellement de nature thiophénique, benzothiophénique ou encore dibenzothiophénique.

Le réacteur de vaporeformage est donc précédé d'un réacteur dit de garde qui permet d'éliminer les impuretés en soufre contenues dans la charge éthanol dénaturé par adsorption sur un solide adsorbant.

Les solides adsorbants qui peuvent être utilisés dans le réacteur de garde sont généralement de type nickel sur alumine (comme par exemple le solide adsorbant AxTrap-405, ou D-1275, commercialisé notamment par Axens).

Ils sont, dans leur phase active (nickel majoritairement à l'état réduit), connus pour être pyrophoriques. Leur manipulation ne peut donc se faire à l'air libre que s'ils ont été préalablement passivés, par exemple avec du CO₂, qu'il conviendra de retirer pour les réactiver préalablement à leur utilisation en désulfuration. Cette activation peut se faire en phase gazeuse, sous gaz inerte comme l'azote, ou sous atmosphère réductrice en présence d'hydrogène, ou bien sous phase liquide en utilisant préférentiellement une charge désulfurée. La température pour réaliser cette opération est préférentiellement comprise entre 100°C et 350 °C, et préférentiellement entre 150°C et 200 °C.

Le réacteur destiné à effectuer la désulfuration de la charge de vaporeformage doit donc être activé par une phase de dépassivation du solide adsorbant qui va libérer le CO₂ et rendre ledit solide adsorbant actif.

La présente invention a essentiellement pour but de trouver une solution à cette phase d'activation du réacteur de garde (ou réacteur de désulfuration de la charge du vaporeformage), en faisant en sorte de préserver de toute pollution par le soufre le réacteur de vaporeformage lui même.

### EXAMEN DE L'ART ANTERIEUR

Selon l'art antérieur, l'activation du solide adsorbant se fait à l'aide d'un flux de gaz inerte (azote par exemple) non soufré et chauffé, par exemple entre 100°C et 250 °C, et typiquement entre 150°C et 200 °C. Ce flux de gaz doit être maintenu pendant des temps relativement longs (4 à 24 heures) et avec des débits importants (entre 200 et 500 Nm³/h/m³ d'adsorbant). Ce type d'opération n'est donc envisageable que si le gaz servant à l'activation est à disposition en quantité suffisante, ce qui n'est pas le cas partout. De plus, cette solution est coûteuse en gaz pur consommé.

Selon l'art antérieur, une deuxième alternative consisterait à pré-activer l'adsorbant et à réaliser son chargement sous atmosphère neutre (azote), mais ce type d'intervention est complexe : utilisation de scaphandre, personnel très spécialisé, équipement spécifique lourd, non disponible partout. Ce type d'opération est délicate et lourde à conduire, notamment pour les unités de grande taille (quand le volume d'adsorbant dépasse le m³).

Une troisième alternative consiste à amener le réacteur contenant le lit de garde prêt à l'emploi, mais cette solution requiert le transport d'un équipement qui peut être très lourd et volumineux : plusieurs centaine de litres, voire plusieurs mètre-cubes.

Par ailleurs, cette solution contraint à avoir un deuxième réacteur pour permuter avec le premier lorsque ce premier réacteur est saturé en soufre. Selon cette alternative, un jeu de vannes est nécessaire pour isoler puis enlever le réacteur contenant le lit de garde avec une vanne en entrée et une en sortie pour le maintenir fermé. Cette solution ne peut donc pas fonctionner économiquement dès lors que les deux réacteurs mis en jeu sont trop volumineux et lourds (supérieurs à quelques tonnes). JP 1275697 A décrit l'emploi de deux réacteurs de désulfuration, le premier étant employé de manière temporaire pendant le startup jusqu'à l'activation du second.

### DESCRIPTION SOMMAIRE DES FIGURES

La figure 1 représente un schéma d'un procédé de préparation de charge de vaporeformage dans lequelle réacteur temporaire de désulfuration et le réacteur permanent de désulfuration sont en série.
La figure 2 représente un schéma du procédé de préparation de charge de vaporeformage selon la présente invention dans lequelle le réacteur temporaire de désulfuration fonctionne sur un circuit en dérivation du circuit normal.

### DESCRIPTION SOMMAIRE DE L'INVENTION

La présente invention peut se définir comme un procédé de prétraitement d'une charge de vaporeformage contenant des composés soufrés. Cette charge doit être désulfurée à moins de 1 ppm pour permettre un fonctionnement correct du réacteur de vaporeformage.

Dans le cadre de la présente invention, la charge hydrocarbonée utilisée comme charge de vaporeformage est généralement de l'éthanol dénaturé contenant moins de 10 ppm de soufre.

La charge peut également être une charge hydrocarbure de type essence ou gazole contenant moins de 10 ppm de soufre.

Le réacteur de vaporeformage est donc précédé d'un réacteur de désulfuration que nous appelons dans la suite réacteur de désulfuration permanent.

Le problème que résout la présente invention est lié au fait que le solide adsorbant permettant la désulfuration dans le réacteur de désulfuration permanent est livré dans un état dit "passivé" et que, pour devenir actif, il doit donc être dépassivé par chauffage thermique.

La solution développée par la présente invention consiste à utiliser la charge de vaporeformage elle même comme vecteur de calories permettant la dépassivation du solide adsorbant contenu dans le réacteur de désulfuration permanent.

Or cette charge étant initialement chargée en soufre, il convient de la désulfurer préalablement dans un réacteur de désulfuration placé en amont du réacteur de désulfuration permanent, que nous appelons réacteur de désulfuration temporaire. Le réacteur de désulfuration permanent est quelque fois appelé également réacteur de désulfuration principal.

Dans la suite du texte les termes de réacteur de prétraitement et de réacteur de désulfuration sont strictement synonymes, puisque le prétraitement en question consiste en une désulfuration.

La présente invention peut donc se définir comme un procédé de prétraitement d'une charge de vaporeformage contenant des composés soufrés, par adsorption desdits composés soufrés par adsorption desdits composés soufrés sur un solide adsorbant, faisant appel à deux réacteurs de désulfuration :
- un réacteur de désulfuration temporaire contenant un solide adsorbant actif,
- un réacteur de désulfuration permanent placé en amont de l'unité de vaporeformage qui contient un solide adsorbant à l'état passivé nécessitant une phase de dépassivation pour être rendu actif,
le réacteur de désulfuration temporaire étant mis hors circuit dès que le solide adsorbant du réacteur de désulfuration permanent est activé, et le volume du réacteur de désulfuration temporaire étant compris entre 1/20 et 1/200 de fois le volume du réacteur de désulfuration permanent, et préférentiellement compris entre 1/50 et 1/200 le volume dudit réacteur de désulfuration permanent.

Selon une première variante du procédé de prétraitement d'une charge de vaporeformage qui n'est pas selon l'invention, ledit procédé comporte deux phases:
- dans la première phase dite d'activation, la charge de vaporeformage est envoyée dans le réacteur de désulfuration temporaire (1010) contenant un solide adsorbant actif, puis dans le réacteur de désulfuration permanent (1003) placé en amont de l'unité de vaporeformage, la durée de fonctionnement du réacteur de désulfuration temporaire (1010) étant telle qu'elle permet au réacteur de désulfuration permanent (1003) de s'activer par dépassivation du solide adsorbant contenu dans ledit réacteur,
- dans la seconde phase, le réacteur de désulfuration temporaire (1010) est déconnecté et la charge de vaporeformage est envoyée directement dans le réacteur de désulfuration permanent activé (1003).

Selon le procédé de prétraitement d'une charge de vaporeformage selon l'invention, ledit procédé comporte trois phases:
- dans la première phase, la charge de vaporeformage est envoyée dans le réacteur de désulfuration temporaire (1010) et la charge désulfurée issue de ce réacteur est stockée dans un ballon (1011),
- dans la seconde phase, la charge désulfurée contenue dans le ballon de stockage (1011) est chauffée dans un échangeur (1002), puis envoyée dans le réacteur de prétraitement permanent (1003), puis est refroidie en sortie du réacteur (1003) dans un échangeur (1004) avant de revenir dans le ballon de stockage (1011), elle effectue cette rotation du ballon de stockage (1011) vers le réacteur de prétraitement permanent (1003) jusqu'à l'activation de ce dernier,
- dans la troisième phase, le réacteur de désulfuration temporaire (1010) est déconnecté, et la charge de vaporeformage est envoyée directement dans le réacteur de désulfuration permanent activé (1003).

Le solide adsorbant utilisé dans le réacteur de désulfuration temporaire et le réacteur de désulfuration permanent est le plus souvent constitué de nickel ou d'un mélange de nickel et d'oxyde de nickel déposé sur un support constitué de silice ou de silice alumine.

La charge de vaporeformage est généralement de l'éthanol dénaturé contenant moins de 10 ppm de soufre.

La charge de vaporeformage peut également être une charge hydrocarbonée de type essence ou gazole contenant moins de 10 ppm de soufre.

### DESCRIPTION DETAILLEE DE L'INVENTION

Le procédé de prétraitement d'une charge hydrocarbonée ou éthanol destinée à alimenter une unité de vaporeformage, objet de l'invention, consiste à utiliser la charge de vaporeformage elle-même comme flux d'activation du solide adsorbant contenu dans le réacteur de désulfuration permanent placé en amont de l'unité de vaporeformage.

Ce réacteur de désulfuration permanent placé en amont de l'unité de vaporeformage est indispensable pour traiter les charges de vaporeformage qu'il s'agisse d'éthanol dénaturé, d'essence ou de gazole qui peuvent contenir jusqu'à 10 ppm de soufre.

Le réacteur de désulfuration permanent est ainsi désigné par opposition au réacteur de désulfuration temporaire qui n'intervient dans le présent procédé que pour assurer l'activation du solide adsorbant contenu dans le réacteur de désulfuration permanent.

Comme la charge de vaporeformage contient du soufre, un réacteur de désulfuration temporaire (1010) est placé en amont du réacteur de désulfuration principal (1003).

Ce réacteur de désulfuration temporaire (1010) a un volume typiquement 20 à 200 fois plus petit que celui du réacteur de désulfuration permanent (1003). Préférentiellement le volume du réacteur de désulfuration temporaire est de 50 à 200 fois plus petit que le volume du réacteur de désulfuration principal (1003).

Le réacteur de désulfuration temporaire (1010) contient un solide adsorbant directement actif et permet de désulfurer sur une durée relativement courte une certaine quantité de charge de vaporeformage.

Ainsi il est possible de disposer d'une quantité de charge de vaporeformage non soufrée pendant par exemple les 4 à 24 heures requises pour l'activation du solide adsorbant contenu dans le réacteur de désulfuration permanent (1003).

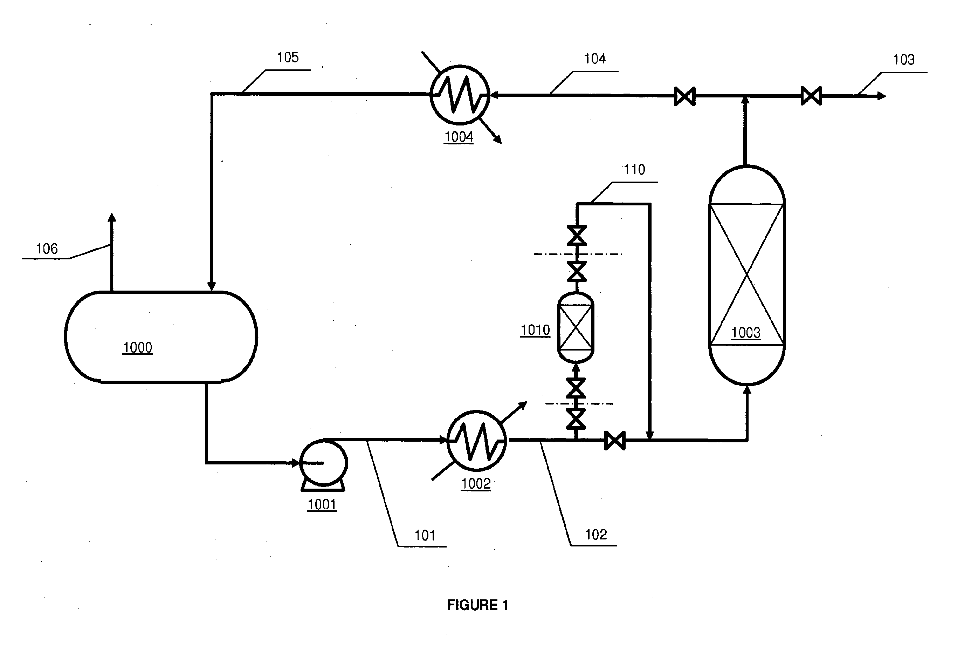

Selon la figure 1 qui représente un procédé de prétraitement d'une charge de vaporeformage qui n'est pas l'objet de l'invention, un jeu de vannes permet à la charge de passer par le réacteur de désulfuration temporaire (1010) via la ligne (110).

Par exemple, pour fixer les ordres de grandeur, pour une unité de production d'hydrogène de 100 Nm³/h d'hydrogène à partir d'éthanol (EtOH) dénaturé contenant 7 ppm de soufre, le réacteur de désulfuration permanent (1003) devra contenir 900 litres de solide adsorbant pour assurer un fonctionnement de l'unité de vaporeformage d'un an avant remplacement dudit solide adsorbant.

Dans ce cas, le réacteur de désulfuration temporaire (1010) placé en amont du réacteur de désulfuration permanent (1003) contient seulement environ 5 litres de solide adsorbant pour permettre la désulfuration de la charge de vaporeformage pendant la phase d'activation de l'adsorbant dudit réacteur de désulfuration permanent (1003).

Le réacteur de désulfuration temporaire (1010) est avantageusement équipé d'un moyen de chauffage électrique de manière à être facilement isolé et même démonté lorsque l'activation de l'adsorbant du réacteur de désulfuration permanent (1003) est terminée.

Selon l'invention, une ligne (104) de recycle de la charge alimente un refroidisseur (1004). En sortie du refroidisseur (1004), la charge ayant servi à l'activation du solide adsorbant retourne à température ambiante via la ligne (105) dans le ballon de charge (1000) où elle est séparée des gaz qui sortiront du ballon de charge (1000) via la ligne de sortie (106).

Ces gaz sont rejetés dans l'atmosphère, tels quels ou préférentiellement après traitement en fonction du type de charge et du site considéré.

La charge désulfurée servant à activer le solide adsorbant contenu dans le réacteur de désulfuration permanent peut être envoyée directement vers le réacteur de reformage via la ligne (103), plutôt que recyclée vers le ballon (1000) via la ligne (104). Après la phase d'activation du solide adsorbant du réacteur de désulfuration permanent (1003), le flux de charge du ballon (1000) peut être dirigé vers l'unité de vaporeformage en fermant, à l'aide d'une vanne, la ligne (104), et en ouvrant la vanne de la ligne (103).

Le réacteur de désulfuration temporaire (1010) peut être aisément démonté de l'installation en raison de sa petite taille, voire être réutilisé sur une autre installation une fois le solide adsorbant pré-activé remplacé sous atmosphère contrôlée en atelier.

L'avantage majeur est de réduire les coûts de mise en service du solide adsorbant du réacteur de désulfuration permanent (1003) sans augmenter significativement les coûts d'investissement.

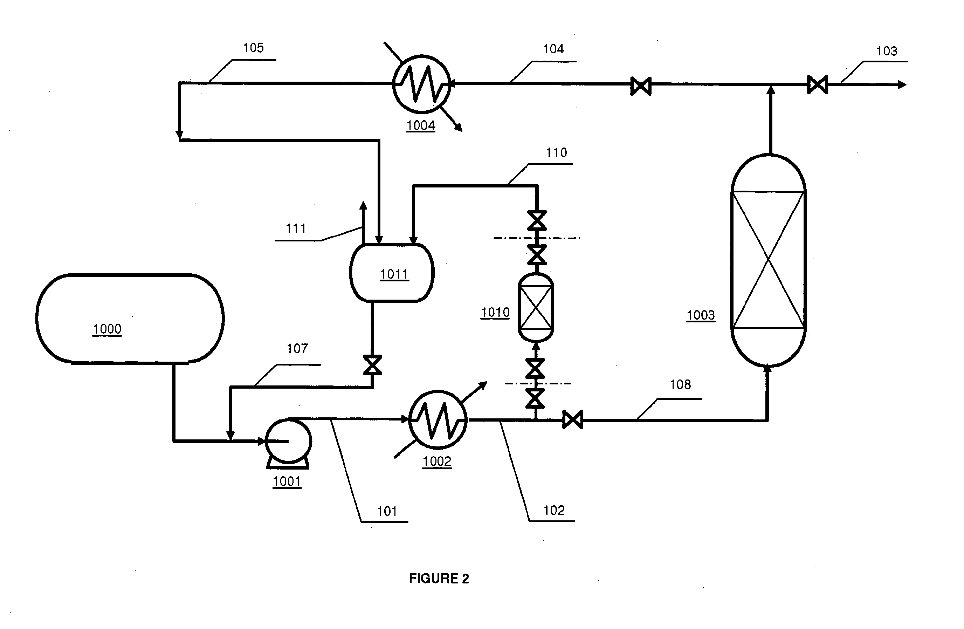

Le procédé de l'invention représenté sur la figure 2, consiste à produire un volume réduit de charge de vaporeformage purifié, représentant par exemple quelques volumes seulement du réacteur de désulfuration permanent (1003), en traitant l'éthanol dénaturé sur le réacteur de désulfuration temporaire (1010).

Ce volume d'éthanol purifié peut alors être utilisé en boucle fermée pour activer l'adsorbant du réacteur de désulfuration permanent (1003).

Par rapport à la configuration précédente, la taille réacteur de désulfuration temporaire (1010) contenant l'adsorbant "pré-activé" peut être diminuée d'un ordre de grandeur.

Un petit volume de charge hydrocarbonée soufré est envoyé du ballon (1000) au travers du réacteur de désulfuration temporaire (1010) via la pompe (1001) et l'échangeur (1002) pour y être purifié.

La charge purifiée est alors stockée dans le petit ballon (1011) muni d'une ligne d'évent (111). Pour activer le lit de garde principal (1003), la charge purifiée est envoyée en boucle du petit ballon (1011) vers le réacteur de désulfuration permanent (1003), via la ligne (107), la pompe (1001), l'échangeur (1002) et la ligne (108).

Durant la phase d'activation, la charge hydrocarbonée qui sort du réacteur de désulfuration permanent (1003) est ensuite renvoyée vers le petit ballon (1011) via l'échangeur (1004) et les lignes (104) et (105).

Lorsque l'étape d'activation est terminée, la charge soufrée contenu dans le ballon principal (1000) est envoyée vers réacteur de désulfuration permanent (1003), via la pompe (1001), l'échangeur (1002) et la ligne (108).

La charge purifiée sortant du réacteur de désulfuration permanent (1003) est alors envoyée par la ligne (103) vers l'unité de vaporeformage en aval.

### EXEMPLES

Cas de Base : On veut produire de l'hydrogène à un débit de 50 Nm³.h⁻¹ pendant une durée de 8000 h (11 mois) à partir d'éthanol dénaturé contenant 2,5 ppm de composés soufrés.

Dans les conditions de fonctionnement de l'unité de vaporeformage, le débit d'éthanol nécessaire est de 32 kg.h⁻¹, soit environ 40 L.h⁻¹.

Pour retirer les composés soufrés de l'éthanol, essentiellement sous forme de thiophène, on utilise le solide d'Axens AxTrap-405, en lit fixe dans le réacteur de désulfuration permanent, fonctionnant à une température de 150°C, dans lequel on fait passer l'éthanol dénaturé à purifier.

Dans les conditions de température et de teneur en soufre, la capacité d'adsorption du solide vis-à-vis des composés soufrés est égale à 0,5 ppm (poids).

Le volume d'éthanol à traiter est de 322 m³, ce qui représente une masse de soufre à retirer de 0,666 kg.

La masse de solide AxTrap-405 à utiliser pour réaliser cette opération est de 129 kg, soit un volume de 161 L.

Ce solide a préalablement été activé, en phase liquide, en utilisant 12 m³ d'éthanol purifié ne contenant pas de soufre, à une température de 150 °C, pendant 8 h. Ce volume représente environ 75 fois le volume du réacteur de désulfuration permanent. Ce volume de 12 m3 d'éthanol purifié (non soufré) représente une quantité d'éthanol difficile à se procurer en raison de la législation qui impose au delà d'un certain volume l'utilisation d'éthanol dénaturé (très probablement soufré) qui en tant que tel est impropre à assurer l'activation optimale du lit de désulfuration permanent.

### Exemple 1 (comparatif):

Dans cet exemple, on produit l'éthanol pur nécessaire à l'activation du réacteur de désulfuration permanent, en traitant l'éthanol dénaturé sur un réacteur de désulfuration temporaire contenant du solide AxTrap-405 déjà activé ex-situ et prêt à l'emploi.

Le volume d'éthanol pur nécessaire pour activer à 150 °C le réacteur de désulfuration permanent est toujours de 12 m³, ce qui représente une quantité de soufre de 0,024 kg, et demande une masse de solide AxTrap-405 de 4,8 kg, soit un volume de 6 L.

Le volume du réacteur de désulfuration temporaire (6 L) est égal à environ 4 % de celui du volume du réacteur de désulfuration permanent.

### Exemple 2 selon l'invention :

Dans cet exemple, on produit l'éthanol pur nécessaire à l'activation du réacteur de désulfuration permanent, en traitant l'éthanol dénaturé sur un réacteur de désulfuration temporaire contenant du solide AxTrap-405 déjà activé ex-situ et prêt à l'emploi.

On utilise l'éthanol pur ainsi produit en boucle, ce qui permet de diminuer le volume nécessaire, et donc de réduire le volume d'éthanol dénaturé à purifier via le réacteur de désulfuration temporaire, et par suite de réduire le volume dudit réacteur de désulfuration temporaire.

On utilise un volume d'éthanol purifié égal à 10 fois le volume du réacteur de désulfuration principal, soit environ 1,6 m³, qui est utilisé en boucle via une recirculation pour activer le réacteur de désulfuration principal.

Ce volume d'éthanol de 1,6 m³ contient 0,003 kg de soufre, ce qui demande d'utiliser 0,6 kg de solide AxTrap-405, soit un volume d'environ 0,8 L.

Le volume du réacteur de désulfuration temporaire est égal à environ 0,5 % du volume du réacteur de désulfuration permanent. Cette variante permet de réduire considérablement le volume du réacteur de désulfuration temporaire ainsi que le volume de l'éthanol dénaturé qu'il faut purifier sur ce réacteur temporaire et qui est utilisé pour activer le réacteur de désulfuration permanent.

## Revendications

1. Procédé de prétraitement d'une charge de vaporeformage contenant des composés soufrés, par adsorption desdits composés soufrés sur un solide adsorbant, faisant appel à deux réacteurs de désulfuration :
- un réacteur de désulfuration temporaire (1010) contenant un solide adsorbant actif,
- un réacteur de désulfuration permanent (1003) placé en amont de l'unité de vaporeformage qui contient un solide adsorbant à l'état passivé nécessitant une phase de dépassivation pour être rendu actif,
le réacteur de désulfuration temporaire étant mis hors circuit des que le solide adsorbant du réacteur de désulfuration permanent est activé, et le volume du réacteur de désulfuration temporaire (1010) étant compris entre 1/20 et 1/200 de fois le volume du réacteur de désulfuration permanent (1003), et préférentiellement compris entre 1/50 et 1/200 le volume dudit réacteur de désulfuration (1003), ledit procédé comportant trois phases:
- dans la première phase, la charge de vaporeformage est envoyée dans le réacteur de désulfuration temporaire (1010) et la charge désulfurée issue de ce réacteur est stockée dans un ballon (1011),
- dans la seconde phase, la charge désulfurée contenue dans le ballon de stockage (1011) est chauffée dans un échangeur (1002), puis envoyée dans le réacteur de désulfuration permanent (1003), puis est refroidie en sortie du réacteur (1003) dans un échangeur (1004) avant de revenir dans le ballon de stockage (1011), elle effectue cette rotation du ballon de stockage (1011) vers le réacteur de désulfuration permanent (1003) jusqu'à l'activation de ce dernier,
- dans la troisième phase, le réacteur de désulfuration temporaire (1010) est déconnecté et la charge de vaporeformage est envoyée directement dans le réacteur de désulfuration permanent activé (1003).

2. Procédé de prétraitement d'une charge de vaporeformage contenant du soufre, selon la revendication 1, dans lequel le solide adsorbant utilisé dans le réacteur de désulfuration temporaire (1010) et le réacteur de désulfuration permanent (1003) est constitué de nickel ou d'un mélange de nickel et d'oxyde de nickel déposé sur un support constitué de silice ou de silice alumine.

3. Procédé de prétraitement d'une charge de vaporeformage contenant du soufre, selon la revendication 1, dans lequel la charge hydrocarbonée utilisée comme charge de vaporeformage est de l'éthanol dénaturé contenant moins de 10 ppm de soufre.

4. Procédé de prétraitement d'une charge de vaporeformage contenant du soufre, selon la revendication 1, dans lequel la charge hydrocarbonée utilisée comme charge de vaporeformage est une essence ou un gazole contenant moins de 10 ppm de soufre.

## Patentansprüche

1. Verfahren zur Vorbehandlung einer Charge für die Dampfreformation enthaltend schwefelhaltige Verbindungen mittels Absorption der schwefelhaltigen Verbindungen auf einem festen Absorbens unter Verwendung zweier Reaktoren der Desulfuration:
- ein temporärer Desulfurationsreaktor (1010) enthaltend ein aktives, festes Adsorbens,
- ein permanenter Reaktor der Desulfuration (1003) angeordnet stromaufwärts einer Einheit zur Dampfreformation der einen festen Adsorber im passiven Zustand enthält, benötigend eine Phase der Depassivierung, um aktiviert zu werden,
der temporäre Desulfurationsreaktor wird außer Betrieb genommen in dem Moment, wo das feste Adsorbens des permanenten Desulfurationsreaktors aktiviert wird, das Volumen des temporären Desulfurationsreaktors (1010) liegt im Bereich zwischen 1/20 und 1/200 des Volumens des permanenten Desulfurationsreaktors (1003), und vorzugsweise zwischen 1/50 und 1/200 des Volumens des besagten Desulfurationsreaktors (1003), wobei das Verfahren drei Phasen umfasst:
- in der ersten Phase wird die Charge für die Dampfreformation in den temporären Desulfurationsreaktor (1010) geleitet und die aus dem Reaktor kommende desulfurierte Charge wird in einem Volumen (1011) gelagert,
- in der zweiten Phase wird die entschwefelte Charge enthalten in dem Lagerungsvolumen (1011) in einem Wärmetauscher (1002) erwärmt, dann in den permanenten Desulfurationsreaktor (1003) überführt, dann am Ausgang des Reaktors (1003) in einem Wärmetauscher (1004) gekühlt, bevor sie in das Speichervolumen (1011) zurückkehrt, wobei diese Rotation vom Lagervolumen (1011) zum permanenten Desulfurationsreaktor (1003) bis zur Aktivierung des Katalysators durchgeführt wird,
- in der dritten Phase wird der temporäre Desulfurationsreaktor (1010) getrennt; und die Charge für die Dampfreformation wird direkt in den aktivierten permanenten Desulfurationsreaktor (1003) geleitet.

2. Verfahren zur Vorbehandlung einer Dampfreformationscharge enthaltend Schwefel gemäß Anspruch 1, bei dem das verwendete feste Adsorbens im temporären Desulfurationsreaktor (1010) und im permanenten Desulfurationsreaktor (1003) aus Nickel oder einer Mischung aus Nickel und Nickeloxiden besteht, die auf einem Trägermaterial aus Silika oder Silikaalumina besteht.

3. Verfahren zur Vorbehandlung einer Charge der Dampfreformation enthaltend Schwefel gemäß Anspruch 1, bei dem die als Charge für die Dampfreformation benutzte kohlenstoffhaltige Charge denaturiertes Ethanol enthaltend wenigstens 10 ppm Schwefel ist.

4. Verfahren zur Vorbehandlung einer Dampfreformationscharge enthaltend Schwefel gemäß einem Anspruch 1, bei dem die als Charge für das Dampfreformationsverfahren verwendete kohlenwasserstoffhaltige Charge ein Treibstoff oder Gasöl enthaltend wenigstens 10 ppm Schwefel ist.

## Claims

1. A process for pre-treating a steam reforming feed containing sulphur-containing compounds by adsorption of said sulphur-containing compounds onto an adsorbent solid, using two desulphurization reactors:
• a temporary desulphurization reactor (1010) containing an active adsorbent solid;
• a permanent desulphurization reactor (1003) placed upstream of the steam reforming unit, which contains an adsorbent solid in the passivated state, necessitating a depassivation phase in order to be rendered active;
the temporary desulphurization reactor being disconnected as soon as the adsorbent solid of the permanent desulphurization reactor has been activated, and the volume of the temporary desulphurization reactor being in the range 1/20 to 1/200 times the volume of the permanent desulphurization reactor (1003), preferably in the range 1/50 to 1/200 of the volume of said permanent desulphurization reactor (1003), said process comprising three phases:
• in the first phase, the steam reforming feed is sent to the temporary desulphurization reactor (1010) and the desulphurized feed coming from this reactor is stored in a drum (1011)
• in the second phase, the desulphurized feed contained in the storage drum (1011) is heated in an exchanger (1002) then sent to the permanent pre-treatment reactor (1003), then is cooled in an exchanger (1004) at the outlet from the reactor (1003) before returning to the storage drum (1011), and said rotation from the storage drum (1011) to the permanent pre-treatment reactor (1003) is carried out until the latter has been activated;
• in the third phase, the temporary desulphurization reactor (1010) is disconnected and the steam reforming feed is sent directly to the activated permanent desulphurization reactor (1003).

2. A process for pre-treating a steam reforming feed containing sulphur according to claim 1, in which the adsorbent solid used in the temporary desulphurization reactor and the permanent desulphurization reactor is constituted by nickel or a mixture of nickel and nickel oxide deposited on a support constituted by silica or silica-alumina.

3. A process for pre-treating a steam reforming feed containing sulphur according to claim 1, in which the hydrocarbon feed used as the steam reforming feed is denatured ethanol containing less than 10 ppm of sulphur.

4. A process for pre-treating a steam reforming feed containing sulphur according to claim 1, in which the hydrocarbon feed used as the steam reforming feed is a gasoline or a gas oil containing less than 10 ppm of sulphur.
